(19) Europäisches Patentamt
European Patent Office
Office européen des brevets

(11) Veröffentlichungsnummer: **0 459 258 A2**

# (12) EUROPÄISCHE PATENTANMELDUNG

(21) Anmeldenummer: **91108084.4**

(22) Anmeldetag: **18.05.91**

(51) Int. Cl.5: **C07B 37/04**, C07C 309/32, C07C 255/50, C07C 47/548, C07D 249/06

(30) Priorität: **31.05.90 DE 4017567**

(43) Veröffentlichungstag der Anmeldung: **04.12.91 Patentblatt 91/49**

(84) Benannte Vertragsstaaten: **CH DE FR GB IT LI**

(71) Anmelder: **BAYER AG**

**W-5090 Leverkusen 1 Bayerwerk(DE)**

(72) Erfinder: **Bader, Axel, Dr.**
**Heymannstrasse 40**
**W-5090 Leverkusen(DE)**
Erfinder: **Arlt, Dieter, Prof. Dr.**
**Rybniker Strasse 2**
**W-5000 Koeln 80(DE)**
Erfinder: **Seng, Florin, Dr.**
**Am Katterbach 46**
**W-5060 Bergisch Gladbach(DE)**

## (54) Verfahren zur Herstellung von Olefin- und Acetylanverbindungen.

(57) Die Arylierung von Olefinen oder Acetylenen mit Arylhalogeniden in Gegenwart von Pd-Phosphan-Komplexen nach der sogenannten "Heck-Methode" erfolgt in einem Zwei- oder Mehrphasensystem, wobei sich der Pd-Katalysator und das Reaktionsprodukt in unterschiedlichen Phasen befinden. Auf diese Weise erfolgt eine einfache Abtrennung und Wiedergewinnung des Palladiums.

EP 0 459 258 A2

Die Synthese von substituierten Olefinen und Acetylenen durch Umsetzung von entsprechenden Halogeniden mit Olefinen und Acetylenen in Gegenwart von Palladium-Katalysatoren und einer Base nach dem Prinzip der "Heck-Methode" (vergl. J. Amer. Chem. Soc. 96, 1133 (1974) sowie US-PS 3 922 299, EP-A 103 544, EP-A 78 768 u.a.m.) ist allgemein bekannt.

Hierbei werden beispielsweise Arylbromide- oder -jodide einphasig in organischen Lösungsmitteln zu Styrol- oder Stilbenderivaten umgesetzt.

Die Aufarbeitung erfolgt durch Destillation oder Umkristallisation des Verdampfungsrückstandes. Über den Verbleib der Palladium-Verbindungen werden dabei keine Aussagen gemacht, insbesondere fehlen Angaben über die Rückgewinnung des wertvollen Edelmetalles.

Aufgabe der vorliegenden Erfindung bestand somit darin, eine Methode bereitzustellen, welche die Nachteile der bekannten Verfahren hinsichtlich der Wiedergewinnung des Katalysators nicht aufweist.

Gegenstand der Erfindung ist deshalb ein Verfahren zur Herstellung von substituierten Olefinen und Acetylenen durch Umsetzung von organischen Halogenverbindungen mit Olefinen bzw. Acetylenen in Gegenwart eines katalytisch wirksamen Palladium-Phosphan-Komplexes und einer Base, dadurch gekennzeichnet, daß man die Umsetzung in einem Zwei- oder Mehrphasensystem durchführt, wobei die Löslichkeiten der Komplexverbindung und des Reaktionsproduktes so zu wählen sind, daß sich diese am Ende der Reaktion in verschiedenen Phasen befinden.

Enthält beispielsweise das Reaktionsprodukt wasserlöslich machende Sulfogruppen, so wird man demzufolge eine solche Pd-Komplexverbindung einsetzen, die in organischen, mit Wasser nicht oder nicht vollständig mischbaren Systemen löslich ist. Umgekehrt verwendet man einen wasserlöslichen Pd-Katalysator, wenn das gewünschte Verfahrensprodukt in Wasser unlöslich ist.

Ein bevorzugter Gegenstand der Erfindung ist ein Verfahren zur Herstellung von Verbindungen der Formel

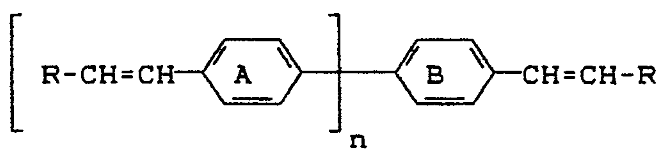

(I)

worin

R H, Alkyl, gegebenenfalls substituiertes Aryl, Hetaryl, CN, COOH oder COO-Alkyl,

n 0 oder 1 bedeuten und

die Ringe A und B weitere Substituenten enthalten können,
durch Umsetzung einer Verbindung der Formel (II)

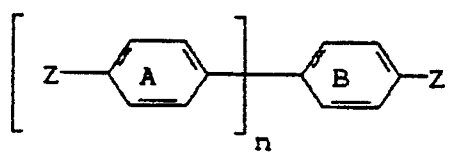

(II)

worin

Z Halogen, vorzugsweise Br oder J, bedeutet,

mit einer Verbindung der Formel

$R\text{-}CH=CH_2$ (III)

worin

R die obengenannte Bedeutung hat.

Als Palladium-Katalysatoren werden Organophosphan-Palladium-Komplexe oder eine Kombination von diese Komplexe bildenden Verbindungen eingesetzt.

Geeignete in wasser unlösliche Palladiumkatalysatoren erhält man durch Kombination von Palladium(O) und/oder Palladium (O)-Verbindungen und/oder Palladium(II)-Verbindungen mit Phosphanliganden der Formel

$PR_3$ (IV)

worin

R Aryl, Alkoxy, Alkyl oder Aryloxy bedeutet.

Pd(O) kann beispielsweise in Form von Pd-schwarz oder Pd/C eingesetzt werden.

Beispiele für Pd(O)-Verbindungen sind $Pd(Ph)_4$ und Bis(dibenzyliden)-palladium($Pd(dba)_2$).

Beispiele für Pd(II)-Verbindungen sind Palladiumhalogenide wie $PdCl_2$ oder $PdBr_2$, Komplexverbindungen der Palladiumhalogenide wie $(PPh_3)_2PdCl_2$ oder $(PhCN)_2PdCl_2$, weiterhin $Pd(CN)_2$, $Pd(NO_3)_2$, Pd-$(OOC_1\text{-}C_{12}\text{-Alkyl})_2$, besonders $Pd(OAC)_2$.

Natürlich ist es auch möglich, vorgebildete Pd-Komplexe der genannten Phosphanliganden einzusetzen.

Geeignete in Wasser lösliche Palladium-Katalysatoren erhält man, wenn man anstelle der oben genannten wasserunlöslichen Phosphane der Formel (IV) wasserlösliche Liganden verwendet. Geeignet sind z.B. Phosphane der Formel

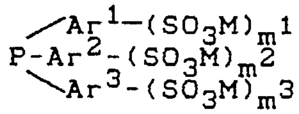
(V)

worin

| | |
|---|---|
| $Ar^1$, $Ar^2$, $Ar^3$ | Aryl, |
| M | ein einfach geladenes Kation und |
| $m^1$, $m^2$, $m^3$ | 0 oder 1 bedeuten, mit der Maßgabe daß $m^1 + m^2 + m^3 = 1$ bis 3 |

oder Phosphane der Formel

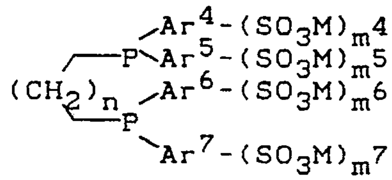
(VI)

worin

| | |
|---|---|
| $Ar^4$, $Ar^5$, $Ar^6$, $Ar^7$ | Aryl, |
| M | ein einfach geladenes Kation, |
| $m^4$, $m^5$, $m^6$, $m^7$ | 0 oder 1 bedeuten, mit der Maßgabe, daß $m^4 + m^5 + m^6 + m^7 = 1$ bis 4 und |
| n | 0, 1 oder 2 bedeutet. |

Natürlich ist es auch hier möglich, vorgebildete Pd-Komplexe der genannten Phosphanliganden einzusetzen.

Die Phosphane der Formeln IV bis VI können im Überschuß eingesetzt werden.

Pro Mol Palladium verwendet man im allgemeinen 2-20 Mol Phosphan der Formeln IV bis VI.

Bei den in den vorstehenden Formeln genannten Arylresten handelt es sich vorzugsweise um Phenylreste, die wiederum weitere Substituenten (z.B. Chlor, Alkyl, Alkoxy, $CF_3$) enthalten können.

Die Reste A und B können außer den vorstehend in Klammern genannten Substituenten durch CN, $SO_3M$, COO-Alkyl, CO-Alkyl, Aryl, Hetaryl, gegebenenfalls durch Alkyl substituiertes Amino substituiert sein. Die Arylreste R können auch für Reste A und B stehen.

Im übrigen befinden sich Pd-Katalysator und Reaktionsprodukt natürlich auch dann in verschiedenen Phasen und lassen sich leicht, z.B. durch Filtration, voneinander trennen, wenn das Reaktionsprodukt im Verlauf der Reaktion ausfällt.

Unter "Alkyl" wird im Rahmen dieser Erfindung vorzugsweise $C_1\text{-}C_4$-Alkyl verstanden. Im Einzelfall (siehe Definition von X) kommen auch langkettige Alkylreste (bis $C_{18}$) in Betracht.

Geeignete Hetarylreste sind Reste von pseudoaromatischen Heterocyclen, wie z.B. Thiophen oder 1,2,3-Triazole, insbesondere solche der Form

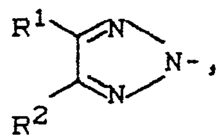

wobei $R^1$, $R^2$ für H, Alkyl, Aryl oder Alkoxy stehen.

Geeignete Kationen M sind Protonen sowie Alkali- und Erdalkaliionen (besonders $Na^+$ und $K^+$). Setzt man die Verbindung der Formel (III), in der R für H steht, mit Verbindungen der Formel (II) um, in der n für O steht, so erhält man Produkte der Formel (I), in der n für O und R für den Rest B steht.

Die Katalysatoren werden im allgemeinen in einer Menge von 0,01 bis 10 Mol-%, bezogen auf die Verbindung (II), verwendet.

Die Reaktions-Temperaturen liegen im allgemeinen zwischen 50 und 200° C, vorzugsweise bei 90 bis 150° C. Es kann von Vorteil sein, die Umsetzungen unter erhöhtem Druck durchzuführen.

Geeignete Basen zur Umsetzung von (II) mit (III) sind tertiäre und cyclische Amine und Alkali- sowie Erdalkalisalze von aliphatischen und aromatischen Carbonsäuren (z.B. Acetate, Propionate, Butyrate, Laurate und Benzoate).

Geeignet sind weiterhin Alkali- und Erdalkalihydroxide, Alkali- bzw. Erdalkalicarbonate sowie Alkalihydrogencarbonate.

Es kann von Vorteil sein, einen Phasentransferkatalysator (PTC) einzusetzen. Geeignete PTC sind z.B.: Tetraethylammoniumchloridmonohydrat, Tetraethylammoniumbromid, Tetraethylammoniumiodid, Tetraethylammoniumtetrafluoroborat, Tetraethylammonium-p-toluolsulfonat, Allyltriethylammoniumbromid, n-Hexyltrimethylammoniumbromid, Phenyltriethylammoniumchlorid, Phenyltrimethylammoniumiodid, Benzyltrimethylammoniumbromid, Benzyltrimethylammoniumiodid, n-Octyltrimethylammoniumbromid, Tetra-n-propylammoniumbromid, Tetra-n-propylammoniumhydrogensulfat, Tetra-n-propylammoniumtrifluoromethansulfonat, Benzyltriethylammoniumchlorid, Benzyltriethylammoniumbromid, Benzyltriethylammoniumtetrafluoroborat, n-Dodecyltrimethylammoniumbromid, Tetra-n-butylammoniumchlorid, Tetra-n-butylammoniumbromid, Tetra-n-butylammoniumiodid, Tetra-n-butylammoniumhydrogensulfat, Tetra-n-butylphosphoniumbromid, Tetraphenylphosphoniumchlorid, Tetraphenylphosphoniumbromid, Tetraphenylphosphoniumiodid, Tetraphnylphosphoniumhexafluoroantimonat, Tetraphenylphosphoniumtetrafluoroborat, N-Hexadecylpyridiniumbromid, Tetra-n-hexylammoniumbromid, Tetra-n-hexylammoniumhydrogensulfat, n-Hexadecyl-tri-n-butylphosphoniumbromid, Triphenylmethyl-triphenylphosphoniumchlorid, Tetra-n-octylammoniumbromid sowie Tetra-n-dodecylammoniumiodid.

Die erfindungsgemäß anzuwendenden Zweiphasensysteme bestehen aus einer wäßrigen Phase und einer mit Wasser nicht oder nicht vollständig mischbaren organischen Phase. Diese organischen Phase kann z.B. ein Lösungsmittel sein.

Beispiele für derartige Lösungsmittel sind aromatische Kohlenwasserstoffe (Toluol, Xylol, Benzol), halogenierte aromatische Kohlenwasserstoffe (Chlorbenzol, Dichlorbenzol), halogenierte aliphatische Kohlenwasserstoffe, (Chloroform, Dichlorethan, Dichlormethan), Dialkyl-, Diaryl- oder Alkylarylether etc..

Die mit Wasser nicht oder nicht vollständig mischbare Phase kann aber auch von einem der Reaktanden gebildet werden, der gegebenenfalls in Überschuß eingesetzt wird. Ein Reaktand kann auch eine weitere, gasförmige Phase bilden.

Ein im Verlauf der Reaktion ausfallendes Produkt bildet ebenfalls eine zweite oder weitere Phase und kann so leicht vom Katalysator abgetrennt werden.

Der Vorteil des neuen Verfahrens besteht - wie eingangs bereits erwähnt - im wesentlichen darin, daß man durch einfache Phasentrennung das Produkt von der Palladiumverbindung abscheiden kann und aus der den Katalysator enthaltenden Phase in üblicher Weise das Edelmetall wiedergewinnen kann.

Die Verfahrensprodukte der Formel (I) sind zum großen Teil bekannt, Bevorzugte Verfahrensprodukte entsprechen der Formel (VII)

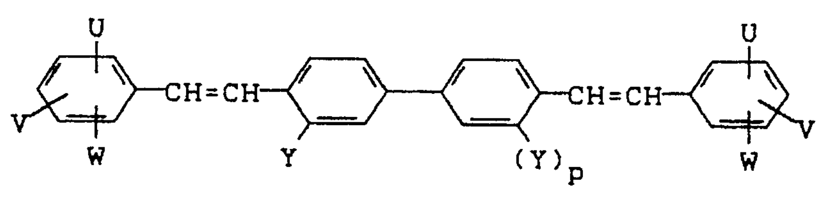

(VII)

worin

p 0 oder 1

Y H oder $SO_3H$ und

U, V, W - unabhängig voneinander H, F, Cl, Methyl, Methoxy, $CF_3$, CN, $OCF_3$, $SO_3H$

bedeuten, und der Formel (VIII)

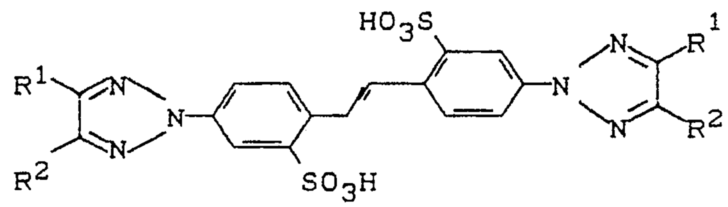

(VIII)

worin

$R^1$, $R^2$ H, Aryl, Aryloxy, Alkyl und Alkyloxy bedeuten.

Die Sulfonsäuregruppen enthaltenden Verbindungen der Formel VII und VIII können selbstverständlich auch in Form der Salze, insbesondere der Alkali (Na, K)-, Amin- und Ammoniumsalze vorliegen.

Die Verbindungen der Formeln (I) bzw. (VII) und (VIII) sind wertvolle optische Aufheller, Fluoreszenzfarbstoffe oder Zwischenprodukte für Aufheller und Farbstoffe.

## Beispiele

### Beispiel 1

Zu 5,16 g (10 mMol) 4,4'-Dibrombiphenyl-3,3'-disulfonsäuredinatriumsalz, 1,6 g (40 mMol) NaOH und 35 mg Hydrochinon in 50 ml Wasser gibt man 2,55 g (25 mMol) Styrol, 45 mg (0,2 mMol) $Pd(OAc)_2$, 100 mg (0,4 mMol) Triphenylphosphin und 0,1 g Benzyltriethylammoniumchlorid in 50 ml Toluol und rührt kräftig 12 h unter Rückfluß. Der ausgefallene Teil des Produktes wird abgesaugt und die Phase im Filtrat getrennt. Aus der wäßrigen Phase wird weiteres Produkt durch Aussalzen mit NaCl gewonnen. So erhält man insgesamt 4,1 g (72 %) 4,4'-Distyrylbiphenyl-3,3'-disulfonsäuredinatriumsalz.

Die den Katalysatorkomplex enthaltende organische Phase kann entweder wiederverwendet oder zur Rückgewinnung des Katalysators (bzw. Pd-Metalls) aufgearbeitet werden.

### Beispiel 2

Zu 18,5 g (0,1 Mol) 4-Brombenzaldehyd, 12,5 g (0,12 Mol) Styrol und 29 ml Tributylamin in 100 ml Toluol gibt man 225 mg (1 mMol) $Pd(OAc)_2$ und 933 mg (0,2 mMol) Di-(m-sulfophenyl)-phenylphosphin in 50 ml $H_2O$ und erhitzt unter kräftigem Rühren 12 h unter Rückfluß.

Die organische Phase wird zweimal mit 1n HCl gewaschen, getrocknet und eingedampft. Nach Umkristallisation aus Cyclohexan erhält man 15.83 g (76 %) Stilben-4-aldehyd vom Fp 109 - 111 °C.

Die den Katalysator enthaltende wäßrige Phase kann entweder wiederverwendet oder zur Rückgewinnung des Katalysators bzw. von Pd aufgearbeitet werden.

### Beispiel 3

Zu 7,36 g der Verbindung

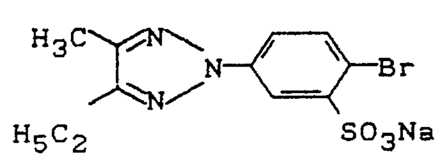

und 1 g NaOH in 50 ml Wasser und 45 mg $Pd(OAc)_2$ und 420 mg Triphenylphosphin in 50 ml Toluol gibt man 0,2 g Benzyltriethylammoniumchlorid und leitet unter kräftigem Rühren und Rückfluß 8 h lang Ethylen

durch die Reaktionsmischung. Der ausgefallene Teil des Produkts wird abgesaugt; die Phasen werden getrennt und aus der wässrigen Phase durch Aussalzen weiteres Produkt gewonnen. Man erhält 5,3 g (88 %) der Verbindung

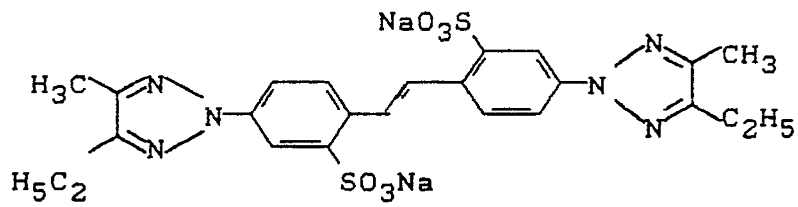

Die den Katalysator enthaltende organische Phase kann entweder wiederverwendet oder zur Rückgewinnung des Katalysators (bzw. Pd-Metall) aufgearbeitet werden.

Beispiel 4

Zu 9,1 g 4-Brombenzonitril, 5,72 g Styrol und 10,2 g Tributylamin in 50 ml Xylol gibt man 56 mg ($Pd(OAc)_2$) und 933 mg Di(m-sulfophenyl)phenylphosphin in 50 ml Wasser und rührt 8 h kräftig unter Rückfluß. Die organische Phase wird 2 mal mit 1N HCl ausgeschüttelt, eingedampft und der Rückstand getrocknet. Man erhält 7,2 g (70 %) 4-Cyanostilben.

Die wässrige Phase wird wiederverwendet (Beispiel 5).

Beispiel 5

Zu 9,1 g 4-Brombenzonitril, 5,72 g Styrol und 10,2 g Tributylamin in 50 ml Xylol gibt man die den Pd-Katalysator enthaltende wässrige Phase aus Beispiel 4 und rührt 8 h kräftig unter Rückfluß. Es wird aufgearbeitet wie in Beispiel 4, man erhält 7 g (68 %) 4-Cyanostilben.

Beispiel 6

In 100 ml Wasser erhitzt man unter $N_2$ 5,16 g 4,4'-Dibrombiphenyl-3,3'-disulfonsäuredinatriumsalz, 3,3 g Natriumacetat, 45 mg $Pd(OAc)_2$, 373 mg Di(m-sulfophenyl)phenylphosphin, 2,55 g Styrol und 35 mg Hydrochinon 12 h auf 120 °C. Der ausgefallene Feststoff wird abgesaugt und getrocknet. Man erhält 4,3 g (76 %) der Verbindung der Formel

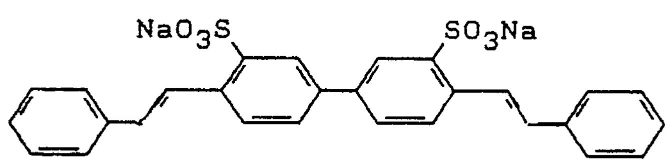

Das Filtrat kann entweder wiederverwendet oder zur Rückgewinnung des Palladiums aufgearbeitet werden.

**Patentansprüche**

1. Verfahren zur Herstellung von substituierten Olefinen oder Acetylenen durch Umsetzungen von organischen Halogenverbindungen mit Olefinen bzw. Acetylenen in Gegenwart eines katalytisch wirksamen Palladium-Phosphan-Komplexes und einer Base, dadurch gekennzeichnet, daß man die Umsetzung in einem Zwei-oder Mehrphasensystem durchführt, wobei die Löslichkeiten der Komplexverbindung und des Reaktionsproduktes so zu wählen sind, daß sich diese am Ende der Reaktion in verschiedenen Phasen befinden.

2. Verfahren gemäß Anspruch 1, dadurch gekennzeichnet, daß man Verbindungen der Formel (I)

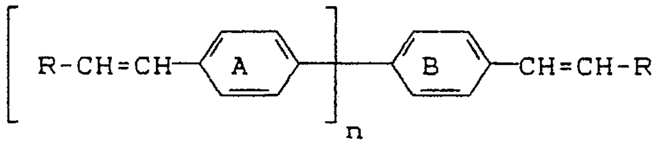

(I)

worin

R H, Alkyl, gegebenenfalls substituiertes Aryl oder Hetaryl, CN, COOH oder COO-Alkyl,

n 0 oder 1 bedeuten und

die Ringe A und B weitere Substituenten enthalten können, durch Umsetzung einer Verbindung der Formel (II)

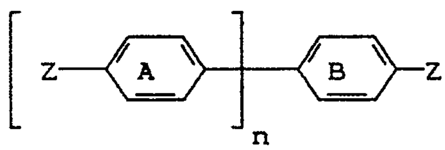

(II)

worin

Z Halogen, vorzugsweise Br oder J, bedeutet,

mit einer Verbindung der Formel

$R\text{-}CH=CH_2$ (III)

worin

R die obengenannte Bedeutung hat, herstellt.

3. Verfahren nach Anspruch 1, dadurch gekennzeichnet, daß man als in Wasser unlösliche Katalysatoren eine Kombination von Palladium(O) und/oder Palladium(O)-Verbindungen und/oder Palladium(II)-Verbindungen mit Phosphanliganden der Formel

$PR_3$ (IV)

worin

R Aryl, Alkoxy, Alkyl oder Aryloxy bedeutet,

verwendet.

4. Verfahren nach Anspruch 1, dadurch gekennzeichnet, daß man als in Wasser lösliche Katalysatoren eine Kombination von Palladium(O) und/oder Palladium(O)-Verbindungen und/oder Palladium(II)-Verbindungen mit Phosphanliganden der Formel (V)

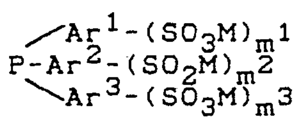

(V)

worin

$Ar^1$, $Ar^2$, $Ar^3$ Aryl,

M ein einfach geladenes Kation und

$m^1$, $m^2$, $m^3$ 0 oder 1 bedeuten, mit der Maßgabe, daß $m^1 + m^2 + m^3 = 1$ bis 3

oder Phosphanliganden der Formel (VI)

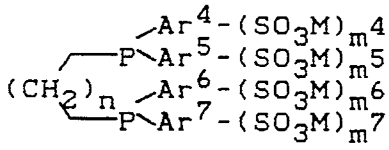

(VI)

worin

| | |
|---|---|
| $Ar^4$, $Ar^5$, $Ar^6$, $Ar^7$ | Aryl, |
| M | ein einfach geladenes Kation, |
| $m^4$, $m^5$, $m^6$, $m^7$ | 0 oder 1 bedeuten, mit der Maßgabe, daß $m^4 + m^5 + m^6 + m^7 = 1$ bis 4, und |
| n | 0, 1 oder 2 bedeutet, verwendet. |